# EUROPÄISCHE PATENTSCHRIFT

(11) **EP 1 302 198 B1**
(45) Veröffentlichungstag und Bekanntmachung des Hinweises auf die Patenterteilung: **15.01.2014**
(21) Anmeldenummer: 02090311.8
(22) Anmeldetag: 30.08.2002
(51) Int. Cl.: A61K 8/97, A61Q 13/00

(54) **Kosmetische Duftzusammensetzung**
Perfumed cosmetic composition
Composition cosmétique parfumante

(30) Priorität: 10.09.2001 DE 10145833
(43) Veröffentlichungstag der Anmeldung: 16.04.2003
(73) Patentinhaber: Coty B.V., 2031 CC Haarlem (NL)
(72) Erfinder: Golz-Berner, Karin, 98000 Monaco (MC); Zastrow, Leonhard, 98000 Monaco (MC); Bernini, Dorothée, 90000 Monaco (MC)
(74) Vertreter: Walter, Wolf-Jürgen

(56) Entgegenhaltungen:
- EP-A- 0 781 544
- EP-A- 1 195 099
- DE-A- 4 204 255
- FR-A- 2 770 776
- US-A1- 2001 001 666
- DATABASE CAPLUS [Online] CHEMICAL ABSTRACTS SERVICE, COLUMBUS, OHIO, US; retrieved from STN Database accession no. 1997:731707 XP002226556 & JP 09 291011 A (KOSEI CO) 11. November 1997 (1997-11-11)
- DATABASE CAPLUS [Online] CHEMICAL ABSTRACTS SERVICE, COLUMBUS, OHIO, US; retrieved from STN Database accession no. 1978:465139 XP002226557 & JP 53 044639 A (OGAWA YOSHIKAZU) 21. April 1978 (1978-04-21)
- DATABASE WPI Week 200123, Derwent Publications Ltd., London, GB; AN 2001-225263, XP002226558 & RU 2 161 950 C (USPENSKAYA) 21 Januar 2001

## Beschreibung

Die Erfindung betrifft eine kosmetische Duftzusammensetzung mit verbesserter Hautwirksamkeit.

Von Parfümzusammensetzungen ist bekannt, daß sie neben essentiellen ölen auch Extrakte von Pflanzen enthalten können. Aus JP 07-102278 A sind Parfüme bekannt, die wasser- und alkohollöslich sind. Sie enthalten eine Hemicellulose und können weiterhin Aromen von Johannisbeeren, Melonen, Bananen und Ananas enthalten sowie essentielle öle von bestimmten Pflanzen.

Weiterhin ist aus der FR 69.41399 bekannt, etherische öle von Ananasblüten in eine Parfümzusammensetzung einzubringen, wobei die Extrakte alkoholische Extrakte sind.

Die Patentanmeldung US 2001/001666 bezieht sich auf Apfelextrakt haltige jedoch gänzlich alkoholfreie Lotionen. Die deutsche Offenlegungsschrift DE 42 40 255 offenbart ein Ölhaltiges Haarwuchsmittel enthaltend unter anderem Apfelöl gewonnen aus der Sorte Red Delicious sowie Granatapfelöl auf pflanzlicher Basis oder Paraffinbasis. Die europäische Anmeldung EP 0781544 verwendet Ethanol ausschliesslich zur Heissextraktion (unter Rückflussbedingungen - im Gegensatz zur Kaltextraktion) und schweigt sich über Ethanol als Inhaltsstoff für Endprodukte aus. Die japanischen Anmeldungen JP 53 044639 (OGAWA YOSHIKAZU) und JP 09 291011 (KOSEI CO) sowie die russische Anmeldung RU2161950 (USPENSKAYA) weisen niedrige Ethanolgehalte auf (5%, bis 26% bzw. 20%) und deuten zwar teilweise an, dass Äpfel mittels einer Kaltextraktion bei 15-35 °C extrahiert werden, allerdings schweigen sie sich über eine Vakuumdestillation aus.

Der Erfindung liegt die Aufgabe zugrunde, eine Duftzusammensetzung bereitzustellen, die trotz hoher Gehalte an Alkohol, insbesondere Ethanol, eine deutlich herabgesetzte oder keine hautirritierende Wirksamkeit hat, zugleich aber attraktiv in Duft und Färbung ist.

Erfindungsgemäß enthält die kosmetische Duftzusammensetzung 0,01 bis 10 Gew-% eines alkoholischen Extraktes aus roten Äpfeln, grünen Äpfeln oder eines Extraktgemisches aus roten und grünen Äpfeln, wobei der Extrakt ein Extrakt der Gesamtfrucht ist und erhältlich ist durch Kaltextraktion bei 15-30°C und nachfolgender Vakuumdestillation bei 15-30°C;
0,01 bis 20 Gew-% eines Parfümöls; und
20 bis 99,98 Gew-% Ethanol und weiteren kosmetischen Hilfs- und/oder Wirkstoffen, wobei der Gehalt an Ethanol im Bereich vom 40-90 Gew% liegt und
wobei alle Angaben auf das Gesamtgewicht der Zusammensetzung bezogen sind.

Es wurde gefunden, daß ein Extrakt aus roten oder grünen Äpfeln, der durch Extraktion mit einem einwertigen niederen Alkohol wie Ethanol oder Isopropanol bei 15-35 EC über einen Zeitraum von 1 bis 36 Stunden unter Bewegung des Extraktionsgemisches gewonnen wurde, eine deutliche antiallergische Wirksamkeit insbesondere bei Einbringen in eine alkoholische Duftzubereitung zeigt.

Es ist bekannt, daß kosmetische Duftzubereitungen, die allgemein Gehalte an Ethanol im Bereich von 40 bis 90 Gew-% haben, wie Parfüms und Rasierwässer, teilweise eine hautirritierende Nebenwirkung bei Anwendern aufweisen. Diese Wirkung kann durch den Zusatz des erfindungsgemäßen alkoholischen Apfelextraktes oder Gemischen davon stark oder vollständig herabgesetzt werden.

Ein bevorzugtes Extraktionsmittel für die Äpfel ist Ethanol. Ethanol ist auch der bevorzugte Trägerstoff für den alkoholischen Extrakt in der kosmetischen Duftzubereitung. Es ist bekannt, daß Duftzubereitungen wie Parfüms klare Lösungen sein müssen. Es wurde gefunden, daß mit normalen Apfelextrakten diese klaren Lösungen nicht erhältlich sind, da dann in jedem Falle feine Eintrübungen im Parfüm enthalten sind. Der für die Erfindung eingesetzte Apfelextrakt ist daher ein solcher, der durch kalte Extraktion (15-35 EC, vorzugsweise 15-22 EC) und nachfolgende mehrfache Vakuumdestillation bei 15 bis 30 EC, vorzugsweise 15-25 EC erhalten wird, wobei der Extrakt danach vollständig klar und frei von jeglichen feindispersen Stoffen ist. Der Extrakt wird gegebenenfalls durch ein Membranfilter filtriert und stellt eine echte Lösung dar, wenigstens jedoch eine kolloidale Lösung.

Weiterhin bevorzugt ist das Vorhandensein eines Apfel-Duftstoffes als Parfümöl. Die durch den Apfelextrakt vorhandene leichte Rot- oder Grünfärbung kann durch alkohollösliche Farbstoffe verstärkt werden. Dadurch kann insgesamt ein typisches "Apfelparfüm" mit unterschiedlicher Färbung zu unterschiedlichen Anlässen kreiert werden.

Eine bevorzugte kosmetische Duftzusammensetzung besteht daher aus einem Extrakt roter Äpfel, einem essentiellen Apfelöl als zusätzlichem Duftstoff und einem roten Farbstoff, wodurch eine klare ethanolische Lösung erhalten wird.

Eine weitere bevorzugte kosmetische Duftzusammensetzung besteht aus einem Extrakt grüner Äpfel, einem essentiellen Apfelöl als zuzsätzlichem Duftstoff und einem grünen Farbstoff, die zusammen eine klare ethanolische Lösung ergeben.

Bevorzugt enthält die erfindungsgemäße Duftzusammensetzung wenigstens 2 Gew-%, vorzugsweise 3-8 Gew-% eines Apfelextraktes und wenigstens 2 Gew-%, vorzugsweise 3-10 Gew-% eines Parfümöls, insbesondere Apfelöl. Der Rest zu 100 % besteht aus Ethanol. Es können auch andere Parfümöle eingesetzt werden. Ein weiterer bevorzugter Gehalt an Parfümöl liegt im Bereich von 7-12 Gew-%.

Eine weitere Ausführungsform der Erfindung besteht darin, daß in der Zusammensetzung der alkoholische Extrakt ein Gemisch von Extrakten aus roten und grünen Äpfeln ist in einem Verhältnis der beiden Extrakte von 10:1 bis 1:10, vorzugsweise 3:1 bis 1:3.

Es wurde weiterhin gefunden, daß der alkoholische Apfelextrakt vorteilhaft eine Hydroxyzimtsäure enthält, insbesondere 0,5 bis 1,2 mg/g Trockenmasse Äpfel an Chlorogensäure, die vermutlich eine stabilisierende Wirkung auf die Blutgefäße ausübt.

Die erfindungsgemäßen Duftzubereitungen entfalten ihre anti-irritative Wirksamkeit insbesondere in alkoholischen Lösungen. Sie können daher bevorzugt als Parfüme, Toilettenwässer, Rasierwässer (Pre-Shaves, After-Shaves), Gesichtswässer, Körperfriktionen (Body splash, Skin freshener) und ähnlichen Produkten mit hohen Alkoholgehalten eingesetzt werden. Es können jedoch auch andere kosmetische Träger verwendet werden, wie solche für Cremes oder Lotionen oder Gele, insbesondere dann, wenn diese ebenfalls Gehalte an niederen Alkoholen erfordern, die dann in Form der alkoholischen Apfelextrakte eingebracht werden.

Die erfindungsgemäße Duftzusammensetzung kann weiterhin alpha-Hydroxysäuren enthalten oder Fruchtsäuren oder Fruchtsäuregemische. Beispiele dafür sind Fruchtsäuren der Zitrone, Pampelmuse, Ananas und Kiwi-Frucht.

Die Erfindung soll nachstehend durch Beispiele näher erläutert werden. Alle Prozentangaben sind Gewichtsprozente, sofern nichts anderes angegeben ist.

### Beispiel 1 Eau de Parfume Pomme

| | |
|---|---|
| Ethanol | 86 |
| Apfelextrakt rot | 3 |
| Apfelöl (Apple oil) | 11 |

Der Apfelextrakt wurde hergestellt durch Behandlung zerkleinerter ganzer roter Äpfel mit Ethanol abs. bei 25 °C für einen Zeitraum von 14 Stunden in einem Extraktionsgefäß. Der filtrierte ethanolische Extrakt wurde einer zweimaligen Vakuumdestillation bei 22-25 °C in einem Rotationsverdampfer unterworfen. Das letzte Destillat wurde direkt für die Zubereitung der Duftzusammensetzung eingesetzt und mit dem Alkohol und dem Duftöl vermischt.

### Beispiel 2 After shave

| | |
|---|---|
| Ethanol | 60 |
| Apfelextrakt grün | 5 |
| Parfümöl | 1,5 |
| Extrapon Hamamalis | 2 |
| Wasser | q.s. ad 100 |

Es wurde wie im Beispiel 1 verfahren, wobei als Ausgangsmaterial zerkleinerte grüne Äpfel eingesetzt wurden.

### Beispiel 3 Shower Gel

| | |
|---|---|
| Tensid (Sulz) | 2 |
| Texapone 40 %ig | 20 |
| Wasser | q.s. ad 100 |
| Apfelextrakt grün | 3 |
| Apfelextrakt rot | 3 |
| Parfümöl | 2 (gebenenfalls + 3,8% Apfelöl) |
| Konservierungsmittel | 0,5 |

Die wie im Beispiel 1 und 2 hergestellten Apfelextrakte wurden mit den restlichen Bestandteilen bei Raumtemperatur (etwa 22 °C) vermischt.

### Beispiel 4 Creme

| **Phase A** | |
|---|---|
| Stearyl Alcohol | 5 |
| Silicone | 3 |

| **Phase B** | |
|---|---|
| Glycerine | 5 |
| Wasser | q.s. ad 100 |
| Carbomer | 0,3 |

| **Phase C** | |
|---|---|
| Triethanolamin | 0,3 |

| **Phase D** | |
|---|---|
| Konservierungsmittel | 0,2 |

| **Phase E** | |
|---|---|
| Apfelextrakt rot | 7 |

| **Phase F** | |
|---|---|
| Apfelöl | 2,3 |

Die Phasen A und B wurden auf etwa 70 °C erhitzt. Unter Rühren wurde die Phase C zugegeben und weiterhin gerührt und dabei auf 35 °C abgekühlt. Nacheinander wurden die Phasen D, E und F bei maximal 35 °C, vorzugsweise darunter zugegeben und das Gemisch gut verrührt.

### Beispiel 5 Vergleichsuntersuchungen

Die anti-irritative Wirksamkeit einer erfindungsgemäßen Duftzusammensetzung wurde an zwei Gruppen von Probanden untersucht:
Eine Zusammensetzung **A1,** bestehend aus 86 % Ethanol, 7 % Apfelextrakt rot und 7 % Parfümöl (Apfelöl),
Eine Zusammensetzung **A2,** bestehend aus 86 % Ethanol, 7 % Apfelextrakt grün und 7 % Parfümöl (Apfelöl),
Eine Zusammensetzung **B**, bestehend aus 86 % Ethanol, 7 % Wasser und 7 % Parfümöl (Apfelöl).

Die Apfelextrakte waren durch nach der Extraktion folgende Vakuumdestillation bei 22-24 °C in vollständig klare Lösungen überführt worden.

Es wurden Probanden ausgewählt, von denen bekannt war, daß sie empfindlich gegenüber Alkohol auf der Haut reagieren. Den 10 Probanden der Gruppe I wurde auf einer Hautfläche von etwa 50 cm² untereinander die Zusammensetzung A1, A2 und B aufgetragen, wobei die Felder durch Streifen voneinander getrennt wurden. Das Ergebnis ist in der folgenden Tabelle aufgeführt, wobei visuelle Betrachtungen der Hautareale einmal täglich bei täglicher Erneuerung der jeweiligen Zusammensetzungen auf den entsprechenden Hautarealen erfolgte.

**Tabelle 1**

| | | Tag 1 | Tag 2 | Tag 3 | Tag 4 | Tag 5 |
|---|---|---|---|---|---|---|
| | A1 | 10a | 9a | 7a | 7a | 7a |
| | | | 1b | 3b | 3b | 3b |
| Gruppe I | A2 | 9a | 7a | 6a | 6a | 6a |
| | | 1b | 3b | 4b | 4b | 4b |
| | B | 2a | 2a | 1a | 0a | 0a |
| | | 8b | 7b | 6b | 6b | 6b |
| | | | 1c | 3c | 4c | 4c |

Die Ziffern vor den Buchstaben bezeichnen die Anzahl der Probanden der jeweiligen Gruppe. Die kleinen Buchstaben bedeuten a) unverändert; b) leichte Rötung; c) mittlere Rötung; d) starke Rötung

**Tabelle 2**

| | | Tag 1 | Tag 2 | Tag 3 | Tag 4 | Tag 5 |
|---|---|---|---|---|---|---|
| | A1 | 9a | 9a | 8a | 7a | 7a |
| | | | 1b | 2b | 3b | 3b |
| Gruppe II | A2 | 10a | 8a | 8a | 8a | 7a |
| | | | 2b | 2b | 2b | 3b |
| | B | 3a | 2a | 2a | 2a | 2a |
| | | 7b | 7b | 7b | 6b | 5b |
| | | | 1c | 2c | 2c | 3c |
| | | | | | 1d | 1d |

Die Auswertung der beiden Gruppen zeigt signifikant bereits am 1. Tag der Anwendung eine anti-irritative Wirkung, die sich auch über mehrere Tage fortsetzt.

## Patentansprüche

1. Duftzusammensetzung, **dadurch gekennzeichnet, dass** sie enthält
0,01 bis 10 Gew-% eines vollständig klaren alkoholischen Extraktes aus roten Äpfeln, grünen Äpfeln oder eines Extraktgemisches aus roten und grünen Äpfeln, wobei der Extrakt ein Extrakt der Gesamtfrucht ist und erhältlich ist durch Kaltextraktion bei 15-35°C und nachfolgender Vakuumdestillation bei 15-30°C;
0,01 bis 20 Gew-% eines Parfümöls; und
20 bis 99,98 Gew-% Ethanol und weiteren kosmetischen Hilfs- und/oder Wirkstoffen, wobei der Gehalt an Ethanol im Bereich von 40 bis 90 Gew-% liegt und
wobei alle Angaben auf das Gesamtgewicht der Zusammensetzung bezogen sind.

2. Zusammensetzung nach Anspruch 1, **dadurch gekennzeichnet, dass** sie als Parfümzusammensetzung vorliegt mit wenigstens 2 Gew-% des Extraktes und wenigstens 3 Gew-% eines Parfümöls und dem Rest zu 100 % mit Ethanol.

3. Zusammensetzung nach Anspruch 1, **dadurch gekennzeichnet, dass** der alkoholische Extrakt ein Gemisch von Extrakten aus roten und grünen Äpfeln ist in einem Verhältnis der beiden Extrakte von 10:1 bis 1:10, vorzugsweise 3:1 bis 1:3.

4. Zusammensetzung nach Anspruch 1, **dadurch gekennzeichnet, dass** das Parfümöl ein Apfelöl ist.

5. Zusammensetzung nach Anspruch 1, **dadurch gekennzeichnet, dass** der alkoholische Extrakt 0,5 bis 1,2 mg einer Hydroxyzimtsäure pro Gramm Trockenmasse Äpfel enthält.

6. Kosmetische Verwendung einer Duftzusammensetzung nach Anspruch 1, wobei der Anteil des Apfelextraktes wenigstens 4 Gew-% beträgt.

## Claims

1. A fragrance composition, **characterized in that** it contains
0.01 to 10 % by weight of a completely clear alcoholic extract from red apples, green apples or of an extract mixture from red and green apples, wherein the extract is an extract from the whole fruit and can be obtained by cold extraction at 15-35°C and subsequent vacuum distillation at 15-30°C;
0.01 to 20°% by weight of a perfume oil; and
20 to 99.98 % by weight of ethanol and further cosmetic auxiliaries and/or active agents, wherein the amount of ethanol contained is in the range from 40 to 90 % by weight and
all percentages relate to the total weight of the composition.

2. The composition according to claim 1, **characterized in that** it is provided as a perfume composition including at least 2 % by weight of the extract and at least 3 % by weight of a perfume oil, the balance to 100 % being ethanol.

3. The composition according to claim 1, **characterized in that** the alcoholic extract is a mixture of extracts from red and green apples, the ratio of the two extracts ranging from 10:1 to 1:10, preferably 3:1 to 1:3.

4. The composition according to claim 1, **characterized in that** the perfume oil is an apple oil.

5. The composition according to claim 1, **characterized in that** the alcoholic extract contains 0.5 to 1.2 mg of a hydroxycinnamic acid per gram of apple solids.

6. The cosmetic use of a fragrance composition according to claim 1, wherein the amount of apple extract contained is at least 4 % by weight.

## Revendications

1. Composition parfumante, **caractérisée en ce qu'**elle contient
0,01 à 10 % poids d'un extrait alcoolisé parfaitement clair de pommes rouges, de pommes vertes ou un mélange d'extraits de pommes rouges et vertes, l'extrait étant un extrait du fruit entier et étant obtenu par extraction à froid à 15-35 °C et par distillation sous vide subséquente à 15-30 °C ;
0,01 à 20 % poids d'une huile parfumée ; et
20 à 99,98 % poids d'éthanol et d'autres substances auxiliaires et agents cosmétiques, la teneur en éthanol se trouvant dans la plage de 40 à 90 % poids et toutes les indications se rapportant au poids total de la composition.

2. Composition selon la revendication 1, **caractérisée en ce que**
elle est une composition parfumante avec au minimum 2 % poids d'extrait et au minimum 3 % poids d'une huile parfumée, et le reste à 100 % avec de l'éthanol.

3. Composition selon la revendication 1, **caractérisée en ce que**
l'extrait alcoolisé est un mélange d'extraits de pommes rouges et vertes, avec une proportion entre les deux extraits de 10:1 à 1:10, de préférence de 3:1 à 1:3.

4. Composition selon la revendication 1, **caractérisée en ce que**
l'huile parfumée est une huile de pomme.

5. Composition selon la revendication 1, **caractérisée en ce que**
l'extrait alcoolisé contient de 0,5 à 1,2 mg d'acide hydroxycinnamique par gramme de matière sèche de pomme.

6. Utilisation cosmétique d'une composition parfumante selon la revendication 1, la part de l'extrait de pomme s'élevant à 4 % poids au minimum.
